Europäisches Patentamt

**European Patent Office**

**Office européen des brevets**

(1) Publication number: **0 297 535**
**A2**

# (12) EUROPEAN PATENT APPLICATION

(21) Application number: **88110368.3**

(22) Date of filing: **29.06.88**

(51) Int. Cl.⁴: **A61K 6/00 , A61K 7/16 , A61K 9/22**

(30) Priority: **30.06.87 US 68251**

(43) Date of publication of application:
**04.01.89 Bulletin 89/01**

(84) Designated Contracting States:
**BE DE FR GB IT NL SE**

(71) Applicant: **VIPONT PHARMACEUTICAL, INC.**
**1625 Sharp Point Drive**
**Fort Collins Colorado 80525(US)**

(72) Inventor: **Southard, G. Lee**
**220 E. Olive Street**
**Fort Collins, CO 80524(US)**
Inventor: **Harkrader, Ronald J.**
**220 E. Olive Street**
**Fort Collins, CO 80524(US)**

(74) Representative: **Vossius & Partner**
**Siebertstrasse 4 P.O. Box 86 07 67**
**D-8000 München 86(DE)**

(54) **Drug delivery devices.**

(57) Drug delivery devices and techniques are described for use in treatment of periodontal disease and the like. The delivery device includes benzo (c) phenanthridine alkaloid in a bioerodable and biocompatible material. The alkaloid is slowly released from the bioerodable material in the oral cavity. The bioerodable material may be polymeric and may be natural or synthetic. The delivery device may be inserted subgingivally (e.g., in a periodontal pocket) where the alkaloid is released slowly over a period of days.

EP 0 297 535 A2

## DRUG DELIVERY DEVICES

This invention relates to devices and techniques for deliverying drugs to a localized site. More particularly, this invention relates to devices and techniques for delivering drugs to a site in the oral cavity. Even more particularly, this invention relates to treatment of periodontal tissues with benzophenanthridine alkaloids.

Periodontal disease is a common and widespread disease which has been shown to be a result of pathogenic bacterial infection established within the gingival sulcus. This condition, if not arrested, will deepen to cause formation of a periodontal pocket. The bacterial found in the periodontal pocket are more anaerobic and contain more gram negative organisms than bacteria found supragingivally.

It is known that several factors prevent or impede the treatment of periodontal disease by supragingival application of drugs or other medicaments. For example, the close proximity of the gum tissue to a tooth impairs diffusion of a medicament into a periodontal pocket. Also, a gingival fluid is continually produced in the pocket and flows outwardly. This fluid flows at a rate of about 1 to 5 microliters per hour in healthy periodontal tissues and at a rate of about 10 to 100 microliters per hour in diseased periodontal tissue.

As a result, the penetration of topically or supragingivally applied medicaments has been largely ineffective in the treatment of periodontal disease. Topical application of medicaments typically does not result in penetration of more than 2 mm. into a periodontal pocket. Since periodontal pockets can be about 5 mm. in depth, topical application does not provide any effective means for treatment.

Systematic application of drugs such as tetracycline to periodontal tissue for treatment of the disease is known. Metronidazole on a film of ethylcellulose has been placed in a periodontal pocket for a period of days during which the active agent is slowly released. Then the film must be removed. It is not bioerodible, see US-A-4,568,535.

Other techniques have also been proposed for treatment of periodontal disease, e.g. capsules or tablets held in the mouth like a throat lozenge, buccal implants, bandages and dressings, topically applied compositions, fibers and dental floss. These techniques, however, are not effective in penetrating the periodontal pocket.

It has also been proposed to insert medicament-impregnated strings or fibers into periodontal pockets to treat the disease; see, for example, US-A-4,406,881 and 4,599,228, incorporated herein by reference. The use of strings or fibers may not result in uniform treatment of the tissue and must be removed by the dentist again at the desired time. These patents also refer to the use of an undiluted paste for treatment of periodontal tissue, the paste being left in place for 10-15 minutes and then removed.

It is also known to use systemic antibiotic treatment to try to control specific pathogenic species. However, use of this treatment results in low concentrations of antibiotic at the site of the periodontal pathogens.

Conventional therapy and treatment of periodontal disease in humans involves the mechanical removal of bacterial plaques and accumulations from the periodontal pocket, often called root planing and scaling. More severe cases may require periodontal surgery to remove damaged tissue. These procedures are expensive, painful, cause extensive bleeding and general discomfort. These procedures are also temporary at best, and frequent recall visits to the dental surgeon are often necessary.

In accordance with the present invention there is provided a bioerodable delivery device which is especially useful in the treatment of periodontal disease. The bioerodable delivery device comprises:

(a) bioerodable, biocompatible material; and

(b) benzo (c) phenanthridine alkaloid.

The alkaloid is releasably contained in the bioerodable material in a manner such that the alkaloid is slowly released from the bioerodable material in the oral cavity.

The delivery device is formable and can be placed into and retained in a periodontal pocket to be treated. The alkaloid slowly releases from the bioerodable material in a manner such that an effective concentration of the alkaloid in the pocket is maintained for a period of several days (e.g., 7-14 days).

The techniques of the invention are useful generally for the treatment of periodontal infections such as periodontitis and irritated gums such as gingivitis. These diseases occur below the gingival margin in the periodontal pocket or along the gum line.

The delivery device bioerodes or bioabsorbs slowly in the periodontal pocket. This allows slow release of the alkaloid into the pocket over a period of several days (e.g., 10 to 14 days). In this manner the alkaloid reduces periodontal pathogenic bacteria (such as Bacteroides gingivalis and many other types of bacteria) and also reduces inflammation.

The bioerodable material is typically polymeric (natural or synthetic). It is biocompatible and preferably is bioabsorbable. It is also non-toxic, non-carcinogenic, and causes no adverse immunologic response. Because the material is bioerodable, the dentist does not have to remove the material after the alkaloid has been released in the periodontal pocket. The amount of alkaloid present in the bioerodable material may vary over a broad range.

The alkaloid may be present in various forms. For example, it may be present as the iminium ion, or as a salt, or as an alkanolamine, alcoholate, or hydroxylate. The alkaloid is an antimicrobial agent, an anti-inflammatory agent, and it also inhibits calcium loss from bone.

The drug delivery devices described herein can be applied to a patient subgingivally by a dental professional using conventional equipment. No strings, fibers or films are used which must be wrapped around a tooth. When the delivery device is in the form of a gel or viscous liquid it may be inserted into the periodontal pocket by means of a syringe. The procedure may be performed rapidly and efficiently. The material penetrates to the bottom of the periodontal pocket so that the anaerobes in the pocket are exposed to the alkaloid as it is released.

Another advantage of the deliver devices of this invention is that they can be provided in any desired physical form. Solid forms also can be made in any size (e.g., to fit a particular pocket size or shape) and are pliable and conformable so that they will conform to the shape of the pocket. The material can be adhesive, if desired, so as to adhere to the tissues. It remains in the periodontal pocket and is not dislodged by crevicular fluid flow.

Also, the use of the alkaloid in the alkanolamine form serves as a controlled release pro-drug.

Other advantages will be apparent from the following detailed description.

The invention is described in more detail hereinafter with reference to the accompanying drawings, wherein like reference characters refer to the same parts throughout the several views and in which:

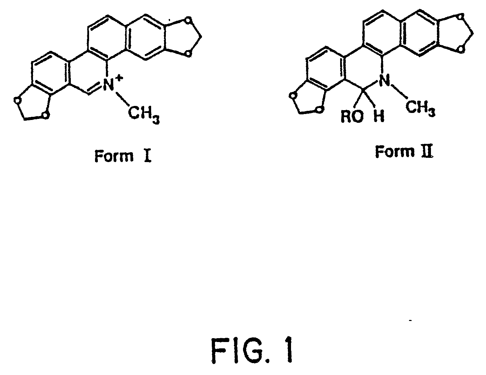

FIGURE 1 shows the iminium ion (Form I) of sanguinarine and also the alkanolamine form (Form II) of sanguinarine;

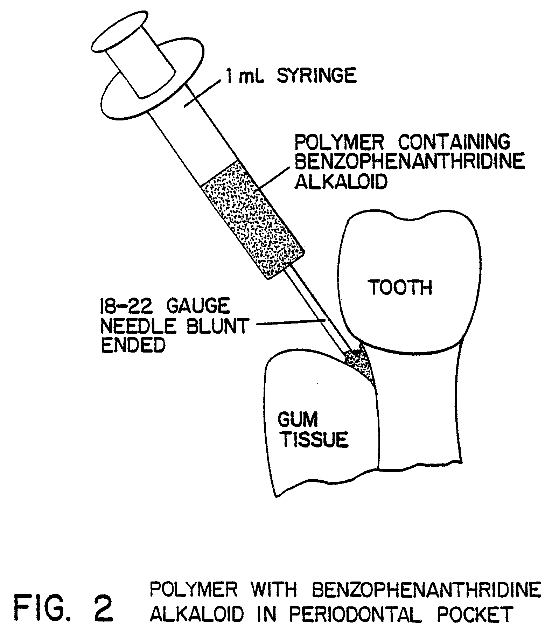

FIGURE 2 illustrates the injection of a gel or viscous liquid form of delivery device into a periodontal pocket adjacent a tooth; and

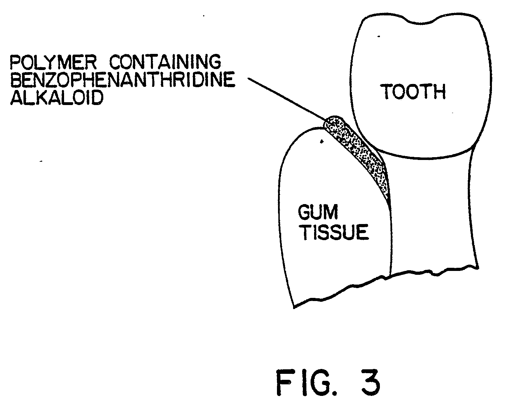

FIGURE 3 shows a solid or semi-solid form of delivery device in a periodontal pocket adjacent a tooth.

The bioabsorbable delivery device of this invention includes bioerodable and biocompatible material and a benzo (c) phenanthridine alkaloid. The delivery device is formable so that it can be placed within a periodontal pocket and will generally conform to the periodontal tissue and the shape of the pocket. The delivery device may be a solid plastic mass or it may be in the form of a gel, viscous liquid or semi-solid. It may also be adhesive so that it will become adhered to the tissue.

The bioerodable material is typically polymeric (natural or synthetic). Preferably it is bioabsorbable in the oral cavity. Representative useful materials include: polylactides; polyglycolides; polycaprolactones; polyanhydrides; pyrollidones (e.g., methylpyrollidone); cellulosic polymers (e.g., carboxymethyl cellulose); methacrylates; collagen (e.g., gelatin), and glycerin. Mixtures and combinations of these may also be used.

particularly useful bioerodable polymers include polylactides having a molecular weight less than about 4000 and an inherent viscosity less than about 0.4 in chloroform at 25°C. Also useful are polylactide/glycolide copolymers having a molecular weight less than about 4000 and an inherent viscosity less than about 0.5 in chloroform at 25°C. Copolymers of polylactide/caprolactone having a molecular weight less than about 4000 are also useful.

The benzo (c) phenanthridine alkaloid may be of various types. For example, useful alkaloids include sanguinarine, sanguirubine, sanguilutine, chelirubine, chelerythrine, and chelilutine. The alkaloid may be in the iminium ion form, or a salt (e.g., sulfate, nitrate, or chloride), or alkanolamine, alcoholate, hydroxylate, acetate, citrate, and tartrate. Mixtures of these may also be used.

The amount of alkaloid present in the delivery device may vary, for example, from about 1-80% by weight. The amount of bioerodable material used may also vary, for example, from about 20-99%. Preferably the alkaloid is present at a concentration of about 1-50%, with a concentration of 10-50% being more preferred. Preferably it is uniformly distributed in the bioerodable material.

The alkaloid slowly releases from the bioerodable material in the oral cavity so as to maintain an effective concentration of the alkaloid in the periodontal pocket for a period of several days. This is much more effective than a simple application of alkaloid alone to the pocket. The amount of alkaloid released in a pocket can be predetermined so that exactly the desired amount is released over the desired time period. In this manner the amount of alkaloid present can be controlled. Assuming an average pocket size of about 250 microliters, it is preferable to place a delivery device therein which contains about 500 micrograms of alkaloid.

Preferably the alkaloid is released from the bioerodable material at a rate in the range of about 30 to 100 micrograms per day. A rate of about 100 micrograms per day is most preferred.

The bioerodable material preferably erodes or is absorbed within about 30 days. Thus, so long as the alkaloid is released in the pocket within about 14 days, it is permissible for the bioerodable material to take a longer period (e.g., up to about 30 days) to be eroded or absorbed.

The bioerodable delivery device of the invention is useful in both animal and human subjects. Examples of animal subjects include dogs, cats and other animals (e.g., monkeys) which may be affected by periodontal disease.

It is preferable to include an antioxidant in the delivery devices. The amount of antioxidant present may vary from about 0.1 to 1% by weight. Useful antioxidants include glutathione, ascorbic acid or other such compounds, e.g. BHT (butylated hydroxy toluene). The presence of antioxidant inhibits the oxidation of alkaloid in the periodontal pocket.

The alkaloid may be soluble or miscible in the bioerodable material, although this is not absolutely required so long as the alkaloid is slowly released from the bioerodable material in the periodontal pocket over a period of about 7-21 days, preferably 10 to 14 days. For example, sanguinarine ethanolate may be dispersed and suspended in glycerine or water and then injected into the periodontal pocket by means of a syringe (e.g., with an 18 or 22 gauge needle).

The alkaloids described herein can be obtained from Papaveraceae plants such as Sanguinaria canadensis, Macleaya cordata, Corydalis sevctvozii, C. ledebouni, Chelidonium majus, Escholtzia, and others. Purification of these alkaloids is described, e.g., in US-A-4,145,412, incorporated herein by reference.

Other isolation procedures require isolation of the alkaloid fraction using a mineral acid and a low molecular weight alcohol (e.g., methanol, ethanol, isopropanol, or other low molecular weight alcohols containing up to 5 carbons), chloroform or methylene chloride, precipitation of the alcohol or solvent fraction using a base, dissolution in water, and precipitation with an acid and a salt such as sodium chloride. The sample is dried and the precipitate is dissolved in an alcohol, ethyl acetate, or methylene chloride and then applied to a silica column. Fractions are eluted with ethyl acetate and methanol (ranging from 0% to 100% methanol in ethyl acetate). The impure alkaloid fractions are precipitated and applied to a Florisil column, eluting with methylene chloride or chloroform. The fractions are recrystallized in a mineral acid and methanol after removing the methylene chloride or chloroform by evaporation.

The sanguilutine and chelerythrine alkaloids are isolated as essentially pure components (i.e., greater than 95% purity). Sanguirubine, chelilutine, and chelirubine require an additional silica clean-up to purify them.

Preparation of sanguinarine is also described in J. Natural Products, Vol. 49, No. 6, pp. 1109-11 (1986), incorporated herein by reference. Sanguinarine chloride is also commercially available from Aldrich Chemical under catalog #30745-9.

It is well known in periodontal literature that the absence of black pigmented bacteroides and spirochetes is associated with periodontal health. An increase in these organisms is associated with periodontal disease. Antimicrobial agents which suppress these microbes have a therapeutic value for the treatment of periodontitis. Previous in vitro studies have shown the following minimum inhibitory concentration (MIC) for anerobes found in the periodontal pocket.

|  | Sanguinarine MIC ($\mu$g/ml) |
|---|---|
| Actinobacillus actinomycetemcomitans | 8-16 |
| Bacteroides gingivalis | 2 |
| Bacteroides melaninogenicas | 8 |
| Bacteroides oralis | 8 |
| Wolinella recta | 4 |
| Eikenella corrodens | 16 |
| Fusobacterium nucleatum | 4 |
| Caprocytophaga gingivalis | 4 |

The drug delivery devices of this invention are useful in controlling these bacteria in the periodontal pocket. The alkaloids also inhibit calcium loss from bone. Further, the alkaloids serve as anti-inflammation agents.

The delivery devices and techniques of the invention are further illustrated by means of the following

4

examples:

## EXAMPLE 1

Sanguinarine chloride (0.2 g) is added to 2.5 g of gelatin which has been dissolved in deionized water. The resulting solution is treated with 2 ml. of phosphate buffered saline and then added at 60°C.. to a solution comprising 20 ml. of petroleum ether and 80 ml. of heavy mineral oil and then stirred vigorously. After the beaker was cooled to 40°C. the solid product was removed and washed with 50/50 petroleum ether/diethyl ether. The gelatin particles are sized through a nylon mesh screen (less than about 25 μm).

A 0.07 g sample of the gelatin was suspended in 50 ml of isotonic saline and held at 37°C. The gelatin was completely dissolved in three days, and the saline solution contained 89 mg /ml or 4,450 mg. of sanguinarine chloride in the 50 ml of saline, as measured by high performance liquid chromatography.

## EXAMPLE 2

A 0.07 g sample containing 98% sanguinarine chloride was added to 2.9 g of USP glycerin. The resulting mixture was added to 100 ml of isotonic saline. The amount of sanguinarine chloride released into the saline was determined by high performance liquid chromatography. The following Table I shows the results obtained.

Table 1

| Time (Hours) | Sanguinarine Chloride (μg/ml in Saline) | Total Sanguinarine Chloride (μg) |
|---|---|---|
| 1 | 33.7 | 3,370 |
| 18 | 149.2 | 14,920 |
| 72 | 198.8 | 19,880 |
| 168 | 274.7 | 27,470 |

## EXAMPLE 3

A 0.06 g sample of sanguinarine ethanolate was prepared by dissolving sanguinarine chloride in ethanol and raising the pH to 8 with ammonium hydroxide. The insoluble precipitate was analyzed by NMR and ultraviolet spectrophotometry and determined to be sanguinarine ethanolate.

The product was then suspended in 2.9 g of USP glycerin and added to a flask containing 100 ml. of isotonic saline. The amount of sanguinarine released into the saline was determined by high performance liquid chromatography. The sanguinarine ethanolate released the sanguinarine into the saline in the iminium ion form. The results are shown in Table 2.

Table 2

| Time (Hours) | Sanguinarine (μg/ml in Saline) | Total Sanguinarine (μg) |
|---|---|---|
| 1 | 3.1 | 310 |
| 18 | 11.1 | 1,110 |
| 168 | 15.4 | 1,540 |

## EXAMPLE 4

A 0.25 g sample of the sanguinarine ethanolate prepared in accordance with Example 3 was mixed with 2.25 g of polycaprolactone diol (mol. wt. 2000; commercially available from Scientific Polymer Products, Inc., Ontario, New York) in methylene chloride. The mixture was stirred under all of the components were in solution, after which the solution was dried under vacuum to remove all of the methylene chloride.

A 0.1 g of the resulting product was placed into a 100 ml sample of isotonic saline. The sanguinarine ethanolate diffused into the isotonic saline as the iminium ion form. The amount of sanguinarine in the saline was determined by high performance liquid chromatography. The isotonic saline solution was changed every 72 hours. The results are shown in Table 3.

Table 3

| Time (Hours) | Sanguinarine (μg/ml in Saline) | Total Sanguinarine (μg) |
|---|---|---|
| 2 | 1.6 | 160 |
| 24 | 2.6 | 260 |
| 48 | 15.6 | 1,560 |
| 72 | 36.0 | 3,600 |
| 120 | 36.8 | 3,680 |
| 240 | 31.2 | 3,120 |

## EXAMPLE 5

A 0.80 g sample of poly-d,1-lactide MW2000 (commercially available from Boerhinger-Ingelheim) was added to methylene chloride with stirring to dissolve the polymer. Then 0.20 g of sanguinarine ethanolate are added to the polymer/methylene chloride solution. After the sanguinarine ethanolate is dissolved the mixture is dried under vacuum to remove the methylene chloride.

A 100 mg sample of the polymer-sanguinarine ethanolate product is placed into a 100 ml sample of isotonic saline at 37° C. The sanguinarine ethanolate is released from the polymer into the saline as the iminium ion form. Analysis of the saline by high performance liquid chromatography showed the results given in Table 4.

Table 4

| Time (Hours) | Sanguinarine (μg/ml in Saline) | Total Sanguinarine (μg) |
|---|---|---|
| 1 | 6.6 | 660 |
| 24 | 18.1 | 1,810 |
| 48 | 14.2 | 1,420 |
| 96 | 13.1 | 1,310 |
| 120 | 14.8 | 1,480 |
| 144 | 17.6 | 1,760 |
| 168 | 23.7 | 2,367 |
| 192 | 60.8 | 6,005 |
| 216 | 62.0 | 6,200 |
| The saline solution was changed every 72 hours. | | |

## EXAMPLE 6

A 0.40 g sample of poly-1-lactide MW 2000 (commercially available from Boerhinger-Ingelheim) and a 0.040 g sample of the polycaprolactone polymer described in Example 4 were mixed in methylene chloride. A 0.20 gram sample of sanguinarine ethanolate was added, after which the methylene chloride was evaporated under vacuum. The dried polymer-sanguinarine ethanolate (0.1 g) was placed in 100 ml of isotonic saline and tested in the manner described in Example 5. The results are shown in Table 5.

Table 5

| Time (Hours) | Sanguinarine (μg/ml in Saline) | Total Sanguinarine (μg) |
|---|---|---|
| 1 | 5.5 | 550 |
| 24 | 47.5 | 4,750 |
| 48 | 73.0 | 7,300 |
| 72 | 87.1 | 8,710 |
| 96 | 19.7 | 1,970 |
| 120 | 40.6 | 4,060 |
| 144 | 69.9 | 6,990 |
| 168 | 19.9 | 1,990 |
| 192 | 31.6 | 3,160 |
| 216 | 34.0 | 3,400 |
| Saline was changed every 72 hours. | | |

## EXAMPLE 7

A 0.2 g sample of poly-1-lactide is dissolved in methylene chloride or chloroform and a 0.80 g sample of sanguinarine ethanolate is added. The resulting solution is evaporated to dryness under vacuum. The polymer/sanguinarine ethanolate (0.1 g is placed into 100 ml of isotonic saline at 37°C. The sanguinarine ethanolate released into the saline as the sanguinarine iminium ion form. Analysis by high performance liquid chromatography showed the concentration of sanguinarine in the saline listed in Table 6.

Table 6

| Time (Hours) | Sanguinarine ($\mu$g/ml in Saline) | Total Sanguinarine ($\mu$g) |
|---|---|---|
| 1 | 5.6 | 560 |
| 24 | 27.5 | 2,750 |
| 48 | | |
| 72 | | |
| 96 | 37.2 | 3,720 |
| 120 | | |
| 144 | | |
| 168 | 83.6 | 8,360 |
| 192 | 97.5 | 9,750 |
| 216 | 131.4 | 13,140 |
| 240 | 134.7 | 13,470 |

## EXAMPLE 7A

High Performance Liquid Chromatography Assay for Benzo (c) Phenanthridine Alkaloid Release from Biodegradable Polymers In Vitro and In Vivo

Samples (0.1 g ) of the polymer containing from 1-80% benzophenanthridine alkaloid by weight were immersed into 100 ml. isotonic saline at 37°C in a water bath. A 5 ml aliquot of the solution was removed periodically and assayed for the alkaloid by the following high performance liquid chromatography (HPLC) method.

A 5CN 10$\mu$ radial pack column (5 mm ID x 100 mm ; from Water Associates) was run at 0.5 ml min with a mobile phase containing 84/16 (u/v) methanol: water with 0.005 M triethylamine and phosphoric acid (pH 4.8). Detection of the benzophenanthridine alkaloid was monitored at 280 nm using sanguinarine in methanol as a standard. After removal of the 5 ml samples, the fluid was replaced with 5 ml of isotonic saline. A 20 $\mu$l sample was injected into the HPLC and analyzed.

The assay for benzophenanthridine alkaloid in gingival crevicular fluid from the bioerodable polymer was taken by an intracrevicular sampling technique using filter paper strips (Harco perio paper). The relative volume of the sample is determined by a change in the dielectric constant of the filter paper (Harco periotron) and the volume was computed from a standard response. Serum is generally used as the standard since gingival crevicular fluid is very close to serum. The amount of benzophenanthridine alkaloid was determined by diluting the sample to 1 ml and analyzing by HPLC.

## EXAMPLE 8

0.100 g of sanguilutine ethanolate was mixed with 0.900 g of polycaprolactone diol (MW2000 Scientific Products, Inc., Ontario, New York) in methylene chloride. The mixture was stirred until all of the components were in solution. The solution was dried under vacuum until all of the methylene chloride had been removed.

A 0.1 g sample of the polymer/sanguilutine ethanolate was placed into 100 ml of isotonic saline at 37°C. The sanguilutine ethanolate released into the saline as the iminium ion form. Analysis of the sanguilutine by high performance liquid chromatography gave the release data shown in Table 7.

Table 7

| Time (Hours) | Sanguilutine (μg/g) | Total Sanguilutine into saline (μg) | % Sanguilutine Released |
|---|---|---|---|
| 1 | 1.7 | 170 | 1.7 |
| 24 | 15.1 | 1,500 | 15.1 |
| 72 | 18.0 | 1,800 | 18.0 |
| 96 | 24.2 | 2,420 | 24.2 |

## EXAMPLE 9

A 0.07 g sample of Sanguinaria Extract containing the benzophenanthridine alkaloids (chelirubine, sanguinarine, sanguirubine, chelerythrine, chelilutine, and sanguilutine) were converted to their ethanolate form· and added to 0.92 g of poly d,1-lactide (MW2000 Boerhringer-Ingerheim) in methylene chloride after all the components had gone into solution. The methylene chloride was then evaporated under vacuum.

A 0.1 g sample of the poly d,1-lactide polymer/Sanguinaria Extract was placed into 100 ml of isotonic saline at 37°C. The extract eluted into the saline as the iminium ion form. Analysis by HPLC showed the following release:

Table 8

| Time (Hours) | Total Alkaloid (μg/ml) | Total Alkaloid (μg) | % Total Alkaloid Release |
|---|---|---|---|
| 1 | 2.9 | 290 | 6.4 |
| 24 | 8.0 | 820 | 17.8 |
| 48 | 7.5 | 750 | 16.7 |
| 72 | 17.2 | 1,720 | 38.2 |
| 96 | 23.1 | 2,310 | 51.3 |
| 120 | 25.3 | 2,530 | 56.1 |
| 144 | 28.0 | 2,800 | 62.2 |
| 168 | 30.9 | 3,090 | 68.6 |
| 216 | 37.1 | 3,710 | 82.2 |

Other variants are possible without departing from the scope and spirit of the present invention.

## Claims

1. A bioerodable delivery device for use in the oral cavity, said delivery device comprising:
(a) bioerodable, biocompatible material; and
(b) benzo (c) phenanthridine alkaloid;
wherein said alkaloid is releasably contained in said material in a manner such that said alkaloid is slowly released from said materials in the oral cavity.

2. A delivery device in accordance with claim 1, wherein said alkaloid is present in an amount of about 1 to 80% by weight and, correspondingly, said material is present in an amount of about 99 to 20% by weight.

3. A delivery device in accordance with claim 1, wherein said biocompatible material is bioabsorbable.

4. A delivery device in accordance with claim 1, wherein said biocompatible material is polymeric.

5. A delivery device in accordance with claims 1 to 4, wherein said biocompatible material is a polylactide, polyglycolide, polycaprolactone, polyanhydride, pyrollidone, cellulosic polymer, methacrylate, collagen, and/or glycerin.

6. A delivery device in accordance with claims 1 to 5, wherein said alkaloid is sanguinarine, sanguirubine, sanguilutine, chelirubine, chelerythrine, and/or chelilutine.

7. A delivery device in accordance with claim 6, wherein said alkaloid is in the iminium ion form.

8. A delivery device in accordance with claim 6, wherein said alkaloid is an alkanolamine, alcoholate, hydroxylate, sulfate, nitrate, chloride, acetate, citrate, and/or tartrate.

9. A delivery device in accordance with claim 6, wherein said alkaloid comprises sanguinarine ethanolate.

10. A delivery device in accordance with any one of claims 1 to 9 for use in a method for treating periodontal disease in a subject comprising applying said device subgingivally to said subject.

11. A method for preparing benzophenanthridine alkanolate comprising the steps of:
(a) dissolving a salt of benzophenanthridine alkaloid in an alcohol having 1 to 8 carbons to form a solution;
(b) raising the pH of said solution to a point wherein said alkanolate precipitates; and
(c) recovering said alkanolate.

12. A method for preparing benzophenanthridine alkanolamine comprising the steps of:
(a) dissolving a salt of benzophenanthridine alkaloid in water containing an alcohol having 1 to 8 carbons;
(b) raising the pH of said solution to a point where said alkanolamine precipitates; and
(c) recovering said alkanolamine.

13. A method for preparing benzophenanthridine hydroxylate comprising the steps of:
(a) dissolving a salt of benzophenanthridine alkaloid in water;
(b) raising the pH of said solution to a point where said hydroxylate precipitates; and
(c) recovering said hydroxylate.

Form I                    Form II

# FIG. 1

1 mL SYRINGE

POLYMER CONTAINING
BENZOPHENANTHRIDINE
ALKALOID

18-22 GAUGE
NEEDLE BLUNT
ENDED

TOOTH

GUM
TISSUE

FIG. 2  POLYMER WITH BENZOPHENANTHRIDINE
ALKALOID IN PERIODONTAL POCKET

POLYMER CONTAINING
BENZOPHENANTHRIDINE
ALKALOID

TOOTH

GUM
TISSUE

FIG. 3